# EUROPEAN PATENT SPECIFICATION

(11) **EP 0 580 071 B1**
(45) Date of publication and mention of the grant of the patent: **10.11.1999**
(21) Application number: 93111295.7
(22) Date of filing: 14.07.1993
(51) Int. Cl.: G01N 33/36, B65H 63/00, B29C 47/92

(54) **Method of production and/or processing as well as winding an endless material of thermoplastic plastic with output of a quality signal as well as a takeup machine**
Verfahren zur Herstellung und/oder Behandlung sowie Aufwicklung eines endlose Materials aus thermopastischem Kunststoff mit Ausgabe eines Qualitätssignals sowie Aufnahmevorrichtung
Méthode de production et/ou traitement ainsique de bobinage d'une matière thermoplastique sans fin avec sortie d'un signal de qualité ainsi qu'un appareil d'enroulement

(30) Priority: 18.07.1992 DE 4223751; 05.09.1992 DE 4229779; 09.12.1992 DE 4241343; 02.03.1993 DE 4306422; 11.06.1993 DE 4319312
(43) Date of publication of application: 26.01.1994
(73) Proprietor: BARMAG AG, D-42862 Remscheid (DE)
(72) Inventor: Binner, Tobias, D-42477 Radevormwald (DE); Schäfer, Klaus Dr., D-42897 Remscheid (DE)
(74) Representative: Kahlhöfer, Hermann, Dipl.-Phys.

(56) References cited:
- EP-A- 0 196 090
- GB-A- 2 243 573
- US-A- 4 351 494
- US-A- 4 514 812
- US-A- 4 758 390
- US-A- 4 843 808

## Description

GB-A-2 243 573 describes a method of producing and/or processing as well as winding an endless material to a package with output of a quality signal according to the preamble of claim 1, wherein the contour of the package is measured and this value is used to find out how the thickness of the endless material has to be changed in order to obtain a package with an adequatly precious cylindrical contour.

The invention relates to a method of producing and/or processing as well as winding an endless material of a thermoplastic substance as defined in the preamble of claim 1 as well as a takeup machine as claimed in claims 32 and 33.

This method is known from EP-A-0 207 471 for texturing a yarn, and characterized in that from a continuously measured product parameter indications relating to the quality of the produced package or produced yarn are obtained for quality parameters which are not directly subject of the measuring.

The known method is successfully applied to textured yarns which are produced by the false twist texturing method.

Endless material in the meaning of the present invention are understood to be yarns, slit-film tapes, films, and foils. These endless materials are characterized in that they are produced continuously or need to be processed continuously, with a plurality of packages being produced one after the other from the continuously advancing strand.

It is the object of the present invention to devise a method as described above, in particular for winding flat yarns, in particular yarns which are spun from manufactured fibers as endless synthetic filaments and wound in the spinning machine, in which a value representative of the quality of the end product is derived continuously, which value permits a classification of the end product and/or a control of the process.

This solution is set forth in the characterizing portion of claim 1.

The invention is based on the recognition that the buildup of the package on which the yarn is wound, is decisively influenced by the quality of the produced yarn such that the condition of the package as defined by one or more of its parameters of state represents a reliable indication as to the quality of the yarn at any instant of the winding cycle (winding of the yarn to a package). The special advantage of the invention in comparison with previously applied methods is that a parameter or the parameters of state of the package reflect on the one hand the course of the process and the actual situation of the process, but on the other hand also the condition of the product. As a result of acquiring a parameter or the parameters of state of the package and their output and evaluation as a quality signal, a binding indication is made with respect to the state of the process and the quality of the product.

In particular it shows that the diameter progression over the entire or predetermined portions of the winding cycle and/or the diameter of the package at certain instants of the winding cycle is representative of an abundance of quality parameters of the yarn and/or the cross-wound package to be produced. It is also possible that derived values, for example, the layer thickness which forms on the tube or package at certain time intervals, the increase in diameter (derivation of diameter as a function of time), the time function of the diameter, coefficients of the time function, and others take the place of the package diameter. It is of advantage to square the value of the diameter as measured and to then draw the first derivative of that value (d²). The advantage is, on the one hand, that by squaring the diameter regularity is improved and that, on the other hand, the derivative of this square is a constant if no irregularity occurs. The first derivative is proportional to the quantity of yarn wound in a given unit of time as long as no disturbances occur. By constant monitoring of this derivative it is, therefore, possible to control the proper functioning of the yarn production from extruder, spinning pump, spinning beam, on the like.

In any event, a comparison occurs with predetermined representative values or representative courses or preset tolerance ranges/-courses (desired values, desired ranges, desired courses). These desired values are predetermined for yarns and cross-wound packages which are acceptable with respect to quality. As aforesaid, it is possible to surround the determined representative values/-courses with tolerance ranges, so that a quality signal is emitted only in the event of a deviation from the tolerance ranges. However, it is also possible to determine the exact consistency of the measured package diameter or the value derived therefrom with the corresponding representative value and to signal any deviation as a quality signal.

With respect to claim 7 and the mean value introduced therein, the following explanation applies:

The mean value is here not understood strictly in its mathematical sense. In practice, a mean value is rather formed in that the parameter of state or the value derived therefrom is supplied to a filter with a predetermined time constant. The selection of the time constant allows to predetermine to which number of measured values the mean value is related. Technically, however, other electronic components are also available to obtain a formation of a mean value, for example, a low-pass filter.

The specific problem arises that in particular in the production and/or processing of yarns of a thermoplastic material very high winding speeds are applied, which amount in texturing machines already to more than 1000 meters per minute, and in spinning machines to more than 3000 meters per minute. The problem of determining the diameter, which arises therefrom as a result of the measuring technique, is advantageously solved at a constant surface speed of the takeup package by claims 21 and 22, and in the case of films and foils by claim 10. An additional or alternative indication can further be obtained from the suggestions of claims 13 and 15. The problems resulting therefrom with respect to the measuring technique are solved without contacting the endless material by claim 14.

A further indicative package parameter to consider is in accordance with claims 16 and 17 the density of the package. The density is here defined as the quotient of the measured weight and the measured package diameter. In so far, this quotient is indicative of the distribution of the yarn mass over the diameter of the package at a constant length of the package, which remains unchanged during the winding cycle.

As previously indicated, it is necessary to produce and/or process endless materials of a thermoplastic substance at a constant speed. This however relates not only to the linear speed of the material, but also to the quantity produced per unit time, which must remain constant. It is obvious that such measurements are very difficult to carry out. The problem is solved by claim 18 in that the package weight is continuously determined, and the course of the weight is observed during the winding cycle.

A suitable measuring method is indicated in claim 19.

A further suitable measuring method of determining the package weight is described in claim 20, by which the weight variation of the entire takeup machine is determined. To this end, it is also possible to measure the torque or the change of the torque which the package exerts with respect to a certain bearing point of the takeup machine.

Such a method may be particularly useful, when the direct determination of the deformation of spindle or spindle bearing is so sensitive that also other disturbances, such as oscillations, superimpose upon the weight-specific deformation, and falsify the measuring result.

The output quality signal may be released as an alarm signal and/or be used for identifying the quality of the produced package and/or be used as a signal for controlling the process. When emitted as alarm signal, the signal may be optical or acoustic or be output as a continuous graphic record. However, it may also be used for identifying the quality of the produced package in that the actually measured values or the values from the comparison of the measured values with the representative value are divided into quality classes and assigned to the respectively obtained product as a quality level. As a process control signal the quality signal may serve again to disconnect the machine or to control the yarn delivery speed, the yarn takeup speed, the spinning speed, and/or other process parameters, which are suitable for correcting the quality parameter which is by experience responsible for the deviation of the determined package diameter or the value derived therefrom from the corresponding representative value. Corresponding methods are subject matter of claims 23-31. These methods are characterized in that they enable an effective, but also sensitive influencing of process, product, and quality during the current process and current production. As previously indicated, the process control of the present invention has the advantage that it allows to correct the production process and/or processing method immediately and without time delay. In the past, this control had to be done by hand, the possibility of intervening directly in the process was nonexistent. Consequently, it was necessary to accept in the meantime an inferior production until a manual control was performed.

Furthermore, there results the possibility of measuring continuously several parameters of state of the package and of controlling different process parameters with each of the parameters of state. There further results as an advantageous alternative possibility that the measured parameter of state or its deviation from the desired value is classified and several process parameters are controlled by classes, for example, that during the production of a yarn with considerable denier deviations, the pump speed is adjusted, and in the event of deviations which are below a certain limit, the withdrawal speed and/or the draw ratio are adjusted.

Further, in multiposition textile machines it is possible to perform a comparison between machines, and to signal the deviation from a predetermined mean value or a predetermined tolerance zone to the mean value. Unless the plurality of the takeup positions are wound in the same timing cycle, a correction with respect to the different starting times of the winding cycle is necessary, since a diameter comparison is useful only when the comparison occurs at identical instants of the winding cycle, i.e., for example, at a certain time interval after the start of the winding cycle.

To carry out the method of the present invention, it is necessary to solve the further problem of making available a takeup machine which cooperates with sensors which detect the selected parameter of state of the packages. Such a takeup machine is defined in claims 32 and 33. Both solutions are novel. A preferred embodiment of such a winding head which is designed and constructed as a weight sensor for the increasing package weight, is set forth in claim 34. This winding head is substantially supported along the line of its center of gravity, in particular mounted for pivotal movement, so that the weight of the winding head itself is balanced and does not influence the measured value determination of the package weight. In this sensor winding head, the measuring device in accordance with claim 35 is arranged preferably stationarily, and the action of force from a torque of the winding shaft with respect to the suspension of the winding head is measured continuously.

Such sensor winding heads allow to provide a spinning system in accordance with claim 36, and a slit-film production line in accordance with claim 37, in each of which a continuous monitoring of production and quality as well as process control for optimizing the product quality is realized.

In the following the invention is described with reference to the drawing, in which
Figure 1 is a schematic view of a spinning system for filament yarns with the processing stages extrusion, drawing, and takeup;
Figure 2 illustrates a yarn traversing mechanism with oppositely rotating blades and a measuring roll arranged on the package for determining the package diameter;
Figure 3 illustrates the takeup portion of the spinning system of Figure 1 with means for determining the package weight;
Figure 4 illustrates the yarn traversing mechanism of Figure 2 with an arrangement of the measuring roll for determining the package weight;
Figure 5 shows the arrangement of Figure 3 for additionally determining and processing a signal indicating the length of the advancing yarn;
Figure 6 shows a spinning system of Figures 1 and 5 with additional determination and processing of a signal for the yarn denier;
Figure 7 shows the takeup portion of the spinning system with a winding head sensor for determining the change in weight of the package during the winding cycle;
Figure 8 shows a further embodiment of a winding head with a weight sensor;
Figure 9 is a schematic view of a slit-film production line with sensor winding heads and process control;
Figure 10 is a schematic view of a wide-slot die with orifice adjustment; and
Figure 11 shows a diagram of yarn tension developed over a predetermined period of time.

Illustrated in the Figures are several embodiments which relate to the spinning of filament yarns. The following description of the spinning installation applies to all Figures. Special characteristics of the present invention, as they result from the individual embodiments, are then described separately.

A filament yarn **1** is spun from a thermoplastic material. The thermoplastic material is supplied via a hopper **2** to an extruder **3**. The latter is driven by a motor **4** which is controlled by a motor control **49**. In extruder **3**, the thermoplastic material is melted. To this end, use is made of the elastic resilience (shearing energy) which is introduced to the material by the extruder. In addition, a heating system **5**, for example, in form of a resistance heater, is provided which is controlled by a heating control **50**. Through a melt line **6** which accommodates a pressure sensor **7** for measuring the melt pressure for a pressure-speed control of the extruder, the melt advances to a gear pump **9** which is driven by a pump motor **44**. The pump motor is controlled by a pump control **45** such that the pump speed is finely adjustable. The pump **9** delivers the melt flow to a heated spin pack **10**, the underside of which accommodates a spinneret **11**. From the latter, the melt exits in form of strands **12** of fine filaments. The strands of filaments advance through a cooling shaft **14**. In the latter an air current **15** is directed by blowing transversely or radially to the web of filaments **12**, thereby cooling the filaments.

At the end of cooling shaft **14**, the web of filaments is combined to a yarn **1** by a spin finish application roll **13** and provided with a liquid spin finish. A delivery roll or godet **16** withdraws the yarn from cooling shaft **14** and spinneret **11**. The yarn loops several times about godet **16**. To this end a freely rotatable guide roll **17** in a crossed arrangement relative to godet **16** is used. The godet **16** is driven at a preadjustable speed by a motor **18** and frequency transmitter **22**. The withdrawal speed is by a multiple higher than the natural speed of exit of filaments **12** from spinneret **11**.

Arranged downstream of godet **16** is a further draw roll or godet **19** with a further guide roll **20**. Both correspond in their arrangement to delivery roll **16** with guide roll **17**. For the drive of draw roll **19** a motor **21** with a frequency transmitter **23** is used. The input frequency of frequency converters **22** and **23** is uniformly preset by a controllable frequency transmitter **24**. In this manner, it is possible to adjust individually the speed of delivery roll **16** or draw roll **19** on frequency converters **22** and **23**. The speed level of draw roll **16** and draw roll **19**, however, is collectively adjusted on frequency converter **24**.

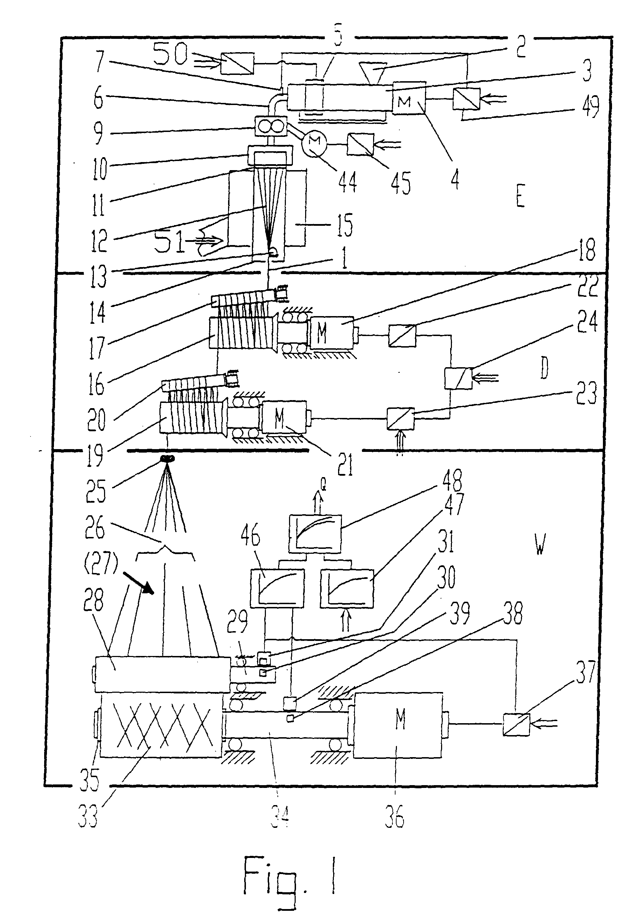

From draw roll **19**, yarn **1** advances to a so-called "apex yarn guide" **25** and thence into a traversing triangle **26**. In Figure 1, a yarn traversing mechanism **27** in accordance with the illustration of Figure 2 is shown. This traversing mechanism employs oppositely rotating blades which traverse yarn **1** over the length of a package **33**. In so doing, the yarn loops, downstream of traversing mechanism **27**, about a contact roll **28**. The contact roll rests against the surface of package **33**, and serves to measure the surface speed of package **33**. The package **33** is formed on a tube **35** secured on a winding spindle **34**. Winding spindle **34** is driven by a spindle motor **36** and spindle control **37** such that the surface speed of package **33** remains constant. To this end, the speed of contact roll **28** freely rotatable about a shaft **29** is scanned as a controlled value by means of a ferromagnetic insert **30** and a magnetic pulse generator **31**.

It should be noted that yarn traversing mechanism **27** may also be a standard cross-spiralled roll with a yarn guide traversing in a cross-spiralled groove over the range of traverse.

In Figure 1, the diameter of package **33** is continuously measured as parameter of state or a value derived from the diameter. Likewise, the weight may be considered as such a value, which is however accompanied by factors of interference, such as density of the wind, enclosed air, crossing angle, and others. Therefore, it is not possible to consider the determined weight as an absolute value but only as a measured value which can be compared with a reference value. To determine the diameter, the speed of spindle **34** and the speed of contact roll **28** lying against the surface of the package are measured. To this end use is made of ferromagnetic inserts **30**, **38** both in spindle **34** and in contact roll **28** as well as corresponding pulse generators **31**, **39**. While the speed of contact roll **28** is simultaneously supplied as controlled value for the adjustment of spindle motor **36** via spindle control **37**, the speed of spindle **34** is also used to control yarn traversing mechanism **27**, which is not described in further detail. In addition, however, both signals are converted into diameter **D** in a computer unit **46**. In a preferred embodiment a multiplier stage may be provided for continuously forming the square of the diameter. Thereafter, the first derivative of the square is formed. As may be seen from Fig. 11 the first derivative of the value(d[D²])/t theoretically has to be a constant. For this first derivative is proportional to the quantity of furnished yarn. But ideally the quantity of yarn produced per unit time in the production of synthetic filaments is absolutely constant.

In Fig. 11, there is depicted a diagram of yarn tension developed over a predetermined interval. A disturbance in the tension of the yarn was deliberately introduced into the diagram, by interference with the winding speed v. In parallel thereto, the first derivative of value (D²) was continuously recorded. As may be seen, as deviations in thread tension occured, the continuity of the first derivative was disturbed in noticeable fashion. By coordinating the deviations in thread tension and deviations in the first derivative D² certain errors in the production process may be determined.
As shown in Fig. 1, the signal representative of the diameter or the signal derived therefrom - as described above - is then supplied simultaneously with a previously stored reference signal from a memory **47** to a comparison unit **48**. The difference signal of comparison unit **48** will then serve as output signal for quality **Q**.

In the embodiment of Figure 3, the weight of package **33** is determined. For this reason, spindle **34** is resiliently mounted in a bearing. The spring-elastic deflection of the bearing is measured. To this end a weight sensor **52** is shown. In practice, the latter includes strain gauges which are arranged on such bearing parts which are deformable as a function of the weight of the package. The course of the weight is compared with a desired value course stored in memory **47**. In a comparison unit **48**, the difference value is formed and the quality signal **Q** is derived therefrom. The quality signal again can be used to influence one or several process parameters in the extrusion (box **E** in Figure 1), or the drawing (box **D** in Figure 1), or even the spindle control **37**.

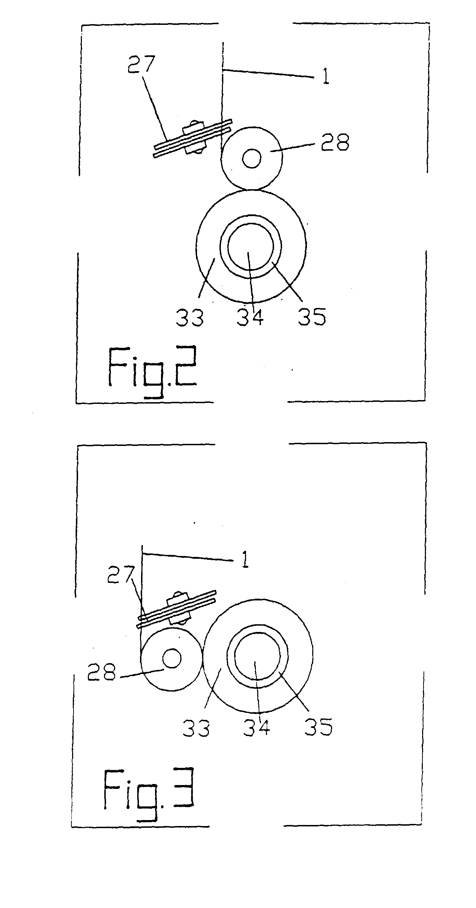

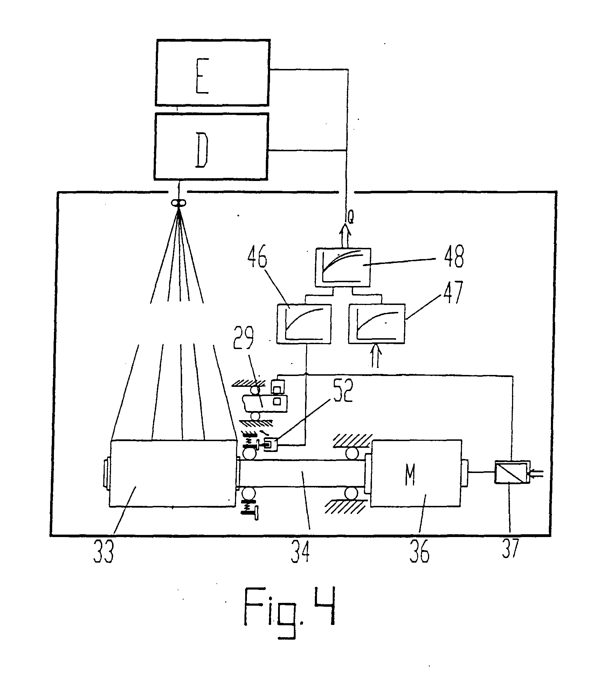

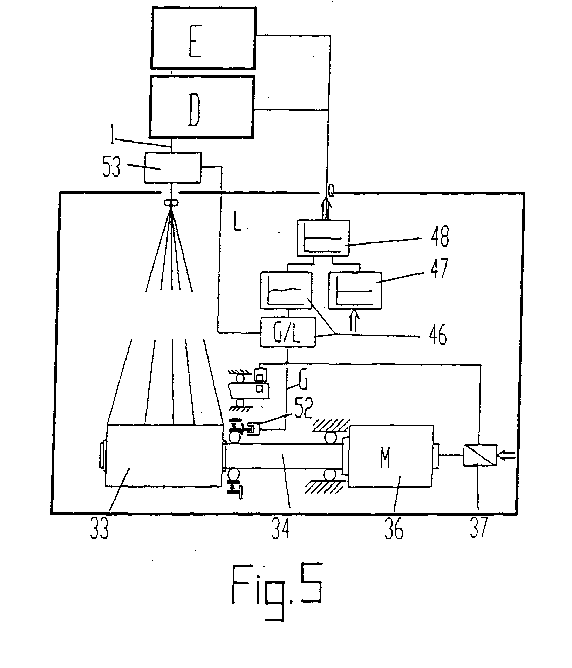

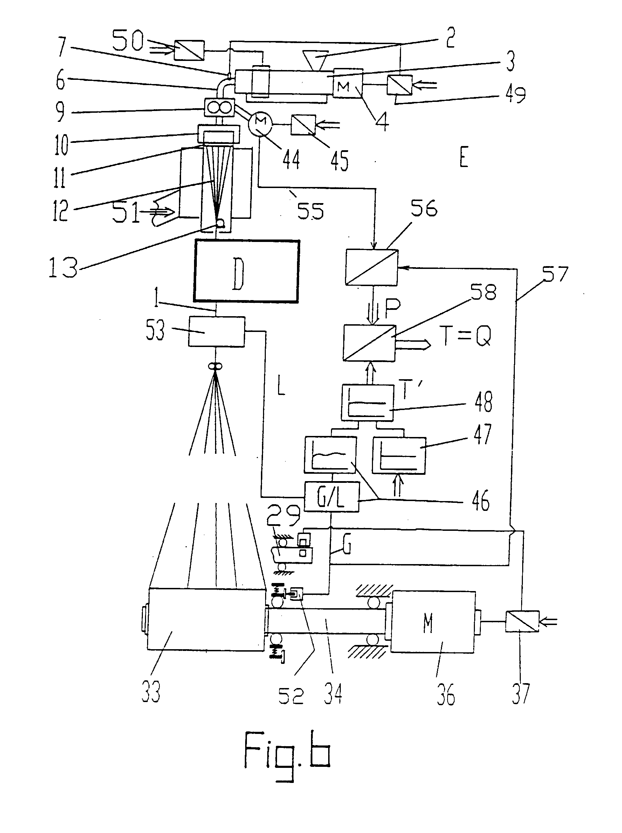

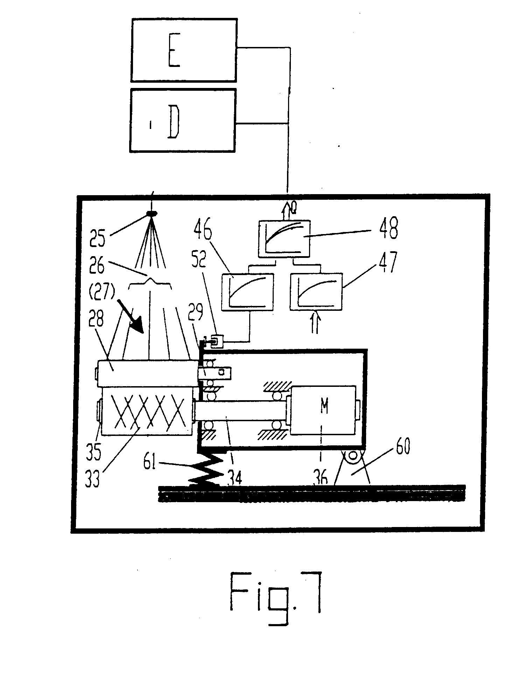

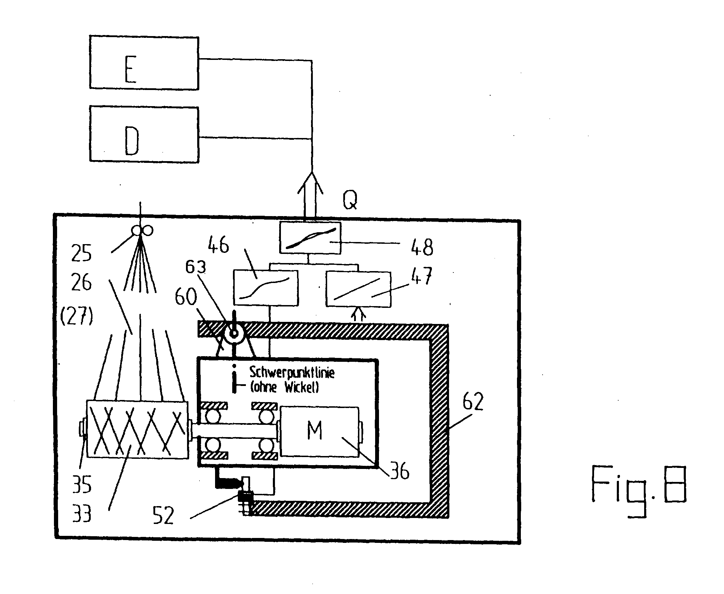

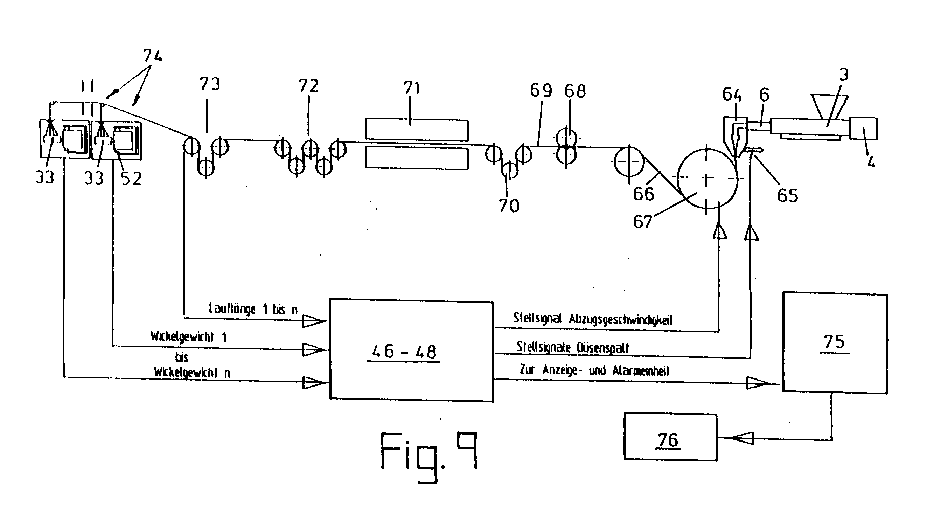

Figure 4 shows a modified arrangement of winding spindle **34** and contact roll **28**, which is preferred when determining the package weight and its change during the winding cycle. In this embodiment, an action of force of contact roll **28** superimposing and possibly falsifying the package weight is compensated in that contact roll **28** is pressed laterally against the surface of package **33**.

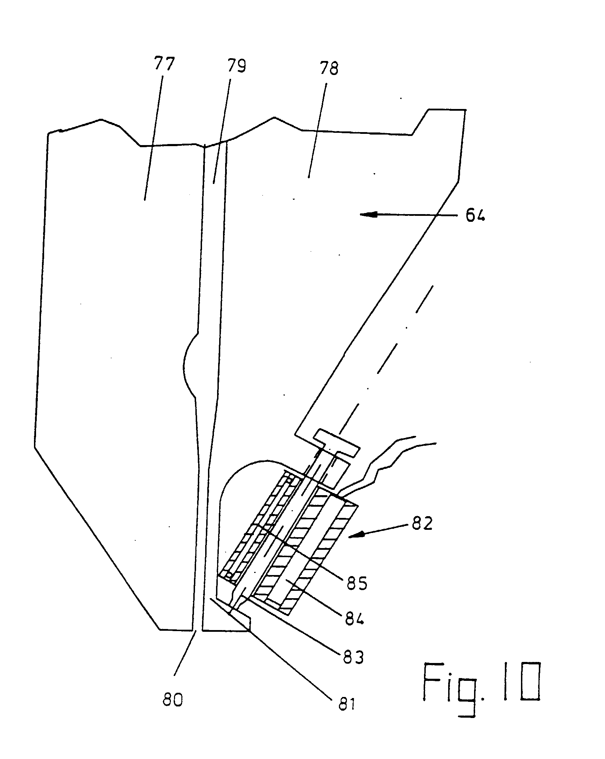

In the embodiment of Figure 5, the denier of yarn **1** on package **33** is continuously measured. To this end, the package weight **G** is continuously measured on the one hand, as to which reference may be made to Figure 3. In addition, the length **L** of the yarn advancing to package **33** is measured by a linear measuring device **53**. The quotient of weight **G** and length **L** of the yarn wound on the package results in the denier. The generated output signal **Q**, as described above, is further processed.

To this end, the actual value from computer **46** is again compared in comparison unit **48** with the desired value input in memory **47**.

Illustrated in Figure 6 is that the denier measurement is corrected in addition by determining the applied spin finish. Spin finish application is here described as the quantity of liquid applied to the yarn. To perform this application, a device **13** (spin finish applicator) is used which is arranged in cooling shaft **14**. To determine the application of the spin finish, the motor **44** of metering pump **9** is provided with a speed sensor not shown. Since the pump is a metering pump in which the discharge is highly speed-dependent, output signal **55** represents the throughput very accurately. Output signal **55** (throughput signal) is supplied to a computer component **56**. The latter also receives in addition via a weight signal **57** the weight **G** which is determined by weight sensor **52**. The difference between the weight determined by weight sensor **52** and the throughput determined by the throughput sensor and integrated over the time results in the actual weight of the spin finish application **P**. The latter as applied per unit time results likewise from the difference of the weight measured per unit time and the time-dependent throughput.

In component **58**, the spin finish application **P** is used to correct the denier value **T'** as is determined by the arrangement of Figure 5. Insofar reference may be made to the description of Figure 5. The output signal of computer component **58** represents then the real denier, namely either the mean value when measured integrated over the time, or the instantaneous value when the respective measurements occur per unit time. This denier **T** again can be used as quality signal **Q**.

In the embodiment of Figure 7, the weight of packages **33** is determined in that the entire takeup machine, hereafter winding head, is mounted in a spring suspension. To this end, on the side facing away from spindle **34** the winding head is mounted for pivotal movement in a swing support **60**, with the pivoting plane extending substantially vertically. On the other side which faces spindle **34** and package **33**, the winding head is supported in a spring **61**. The latter is sufficiently strong, so that the weight increase of package **33** effects only a slight deflection which has no effect on the yarn path geometry.

The deflection of the winding head is picked up by a measuring device **52** and supplied via computer unit **46** and desired value memory **47** to comparison unit **48**. This comparison unit generates the quality signal **Q**. The quality signal again can be used to influence one or several process parameters in the extrusion (box E of Figure 1), or the drawing (box D of Figure 1), or even the spindle control.

The quality signals **Q** which have been generated, as previously described, by the takeup device, are further processed as follows: the quality signal **Q** may be output as optical or acoustic alarm or as a graphic record. The latter allows to mark and classify the produced package **33**.

Alternatively or additionally, the quality signal **Q** may then be supplied to one or several of the control means **23** for draw roll **19** to influence the drawing; to
**24** to control the withdrawal speed;
**45** to control the pump speed;
**49** to control the extruder speed;
**50** to control the heater; or
**51** to control the cooling device.
The extruder control **49** is activated in particular when no metering pump **9** is used. In this instance, the extruder **3** functions as a pump and the activation of extruder control **49**, i.e., the rotational speed of the extruder, allows to influence the output of extruder **3**.

When a metering pump **9** is used, the throughput quantity may be influenced by spin pack **10** and spinneret **11** by activation of pump control **45**, i.e., the rotational speed of metering pump **9**.

The activation of cooling air control **51** allows to influence cooling. This has an effect on the yarn denier **T**. In particular, it is also possible to influence the uniformity of the individual filaments with the use of special cooling devices which allow to cool the webs of filaments **12** and/or spinneret **11** in sectors.

In the embodiment of Figure 1 all parts of the system which are necessary to produce yarn **1**, i.e., to extrude the thermoplastic material, are combined in a box indicated at "**E**". The individual parts of the system are possibly exchangeable. Accordingly in this event other parameters are controlled. In particular, it is possible to replace extruder **3** with a discharge pump, and likewise various other possibilities of cooling the filament web are given.

Shown in the embodiment of Figure 1 is that the produced yarn **1** is drawn by godets **16** and **19**. All elements which in the embodiment are used for drawing, are combined in the box indicated at "**D**". In the present high-speed spinning process, drawing by godets may also be eliminated. In this instance, the yarn **1** is withdrawn from spinneret **11** either by a single godet and advanced to the winding head, or it is withdrawn directly from the spinneret by the takeup device. On the other hand, drawing may also occur by further methods, so that in this instance, block "**D**" would have to be replaced or supplemented with further elements, for example, an additional godet, a heater, in particular a heating tube.

In the illustrated embodiment, the quality parameter **Q** may be used to activate delivery control **24** and/or draw ratio control **23**.

The activation of delivery control **24** allows to influence the speed of godet **16** and godet **19** without changing the speed ratio. In this instance, the draw ratio remains constant, whereas the yarn speed changes. As a result, the denier **T** can be influenced.

The activation **37** of draw ratio control **23** allows to influence the speed ratio between godets **16** and **19**, and to thus change the draw ratio. The change of the draw ratio allows to vary the strength conditions of the yarn, but also the denier.

Finally, the spindle control allows to also control by means of quality parameter **Q** the circumferential speed of package **33** which is regulated by contact roll **28**. This results in particular in an influence on the package buildup and the yarn tension under which the yarn is wound on the package.

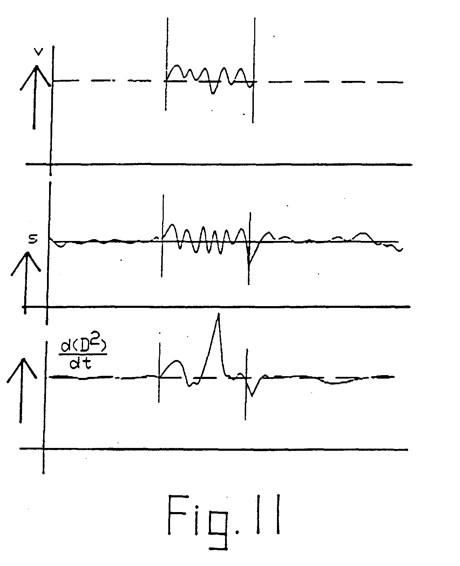

Figure 8 illustrates a winding head **74** of a spinning system or a slit film production line in accordance with Figure 9, which is modified in comparison with Figure 7. In this embodiment, the entire winding head **74**, consisting of winding spindle **34**, bearing and drive motor **36**, as well as contact roll **28** and yarn traversing mechanism **27** is pivotally supported along the line of its center of gravity, i.e. a vertical line through the center of gravity of winding head **74** without package **33** held thereon. The pivot bearing **63** of swing support **60** is arranged in a frame structure **62** such that the torque caused by the forming package **33** loads weight sensor (weighing cell) **52** stationarily arranged on frame structure **62** with respect to axis of rotation **63** of pivot frame **62**, which extends vertically to winding spindle **34**. The evaluation of the weight signals supplied by weight sensor **52** occurs as in Figure 7 and causes quality signals **Q** which are used to control the spinning and processing operations.

Figure 9 is a schematic view of a system for producing slit films, for example, slit-film yarns for carpet backing, braids, binder twine, or the like. Also in this embodiment, the invention is realized with the use of takeup devices constructed as sensor winding heads **74** in accordance with Figure 7 or Figure 8, as is described below.

The thermoplastic plastic which is plasticized in extruder **3** by friction and additional heating, is extruded through melt line **6** and a subsequent wide slot-die **64** with orifice adjustment **65** to a flat film web, and withdrawn by a speed-adjustable cooling roll **67**, and solidified by intensive cooling. Subsequently, the film web **66** passes through a cutting device **68**, where it is split into a web of slit films **69**. The latter is then drawn collectively through a first draw zone **70** with heat setting and a further draw zone **73**, heat set and adjusted in its shrinkage. Finally, all slit films of web **69** are individually wound to cross-wound packages **33** on winding heads **74** with weight sensors in accordance with Figure 7 or 8.

The winding weights of packages **33**, which are determined continuously or in timed fashion by the individual sensors **52** of winding heads **74** are input together with the length of advance of the slit-film web **69** as determined at delivery system **73** at the end of the shrinkage zone, into a central computer unit **46** and compared therein with the course of a representative desired value curve. The quality signals which are output by this unit, appear optically and, if need be, acoustically on a display and alarm unit **75**, and trigger an alarm, when unacceptable deviations from the predetermined tolerance range occur. With a suitable programming of the evaluation unit, however, they are also output already when the tendency to such an unacceptable deviation becomes apparent, so as to warn the operating personnel as early as possible and to counteract these tendencies. However, the quality signals are also output as a graphic record on a printer **76** connected to display unit **75**. Finally, the quality signals are used to control the process, namely to control the delivery speed the withdrawal by cooling roll **67**, provided the signals of all package weight sensors **52** move in direction of an unacceptable deviation from the desired value or a predetermined tolerance range. However, when these signals fail to indicate a uniform tendency, but exhibit tendencies which possibly deviate in groups among each other, the quality signals will be used for adjustment **65** of the die orifice, so as to correct the thickness of the slit films in partial ranges of the extruded film web.

Finally, shown in Figure 10 is a wide-slot die **64** with an adjustment **65** of the die orifice, as is used in the extrusion line of Figure 9. Such dies are known and commercially available. Figure 10 shows the basic setup of such a flat film die **64**. It consists of two halves **77** and **78**, which form between them a melt duct **79** with a melt distribution over the width of die orifice **80**. The one die half **77** is constructed substantially rigid, whereas the second die half **78** is provided with a flexible lip **81** as a result of a cross-sectional narrowing. This lip is divided, when viewed over its length, into several adjustment blocks which cooperate each with associated adjustment devices **82**, and which are controlled by adjustment **65** of the die orifice as a function of determined quality signals.

The adjustment devices **82** consist of expansion pins **83** which are unilaterally held in second die half **78** and supported with their free end on the adjustment blocks of lip **81**. The expansion pins **83** may be heated by heating cartridges **84** and are thereby lengthened, they may be cooled by cooling bores **85** and are thereby shortened, so that the length of the expansion pins is thermally changed, as need arises. As a result, force is introduced to die lip **81** in sections such as the die orifice adjustment requires it for this associated section. It should further be noted that this adjustment can be only a finest adjustment of die orifice **80** in a range from few 1/100 mm up to several 1/10 mm, whereas a manual coarse adjustment must previously be made by the operating personnel at the startup of the line. The described measures and the control of the line by determined quality signals allow to provide for a substantially automated operation with simultaneous product monitoring and quality control.

### NUMERAL REFERENCE LIST

- 1: Filament yarn
- 2: Hopper
- 3: Extruder
- 4: Motor
- 5: Heating system
- 6: Melt line
- 7: Pressure sensor
- 9: Pump, gear pump
- 10: Spinning head, spin pack
- 11: Nozzle, spinneret
- 12: Filaments, strand of filaments
- 13: Spin finish application roll, spin finish applicator
- 14: Cooling shaft
- 15: Blowing, air current
- 16: Delivery roll, godet
- 17: Guide roll
- 18: Drive motor
- 19: Draw roll, godet
- 20: Guide roll
- 21: Drive motor
- 22: Frequency transmitter
- 23: Frequency transmitter, draw ratio control
- 24: Withdrawal control, controllable frequency transmitter
- 25: Apex yarn guide
- 26: Traversing triangle
- 27: Yarn traversing mechanism
- 28: Contact roll
- 29: Shaft of contact roll
- 30: Ferromagnetic insert
- 31: Pulse generator
- 33: Package
- 34: Spindle
- 35: Winding tube
- 36: Drive motor
- 37: Spindle control
- 38: Ferromagnetic insert
- 39: Pulse generator
- 40: Computer unit
- 41: Set-point adjuster
- 42: Comparison circuit
- 44: Pump motor
- 45: Pump control
- 46: Computer unit
- 47: Memory
- 48: Comparison unit
- 49: Extruder control
- 50: Heater control
- 51: Cooling control
- 52: Weight sensor
- 53: Linear measuring device
- 55: Throughput signal
- 56: Computer component
- 57: Weight signal
- 58: Computer component
- 60: Swing support
- 61: Spring
- 62: Frame, frame structure
- 63: Axis of rotation, pivot bearing
- 64: Wide-slot die
- 65: Adjustment of extrusion orifice
- 66: Film web
- 67: Withdrawal by cooling roll
- 68: Cutting device
- 69: Web of slit-film tapes
- 70: First draw zone
- 71: Hot air zone
- 72: Second draw zone with heat setting
- 73: Delivery system
- 74: Winding head, sensor winding head
- 75: Display unit with alarm
- 76: Printer
- 77: Die half (rigid)
- 78: Die half
- 79: Melt duct
- 80: Die orifice
- 81: Die lip
- 82: Adjusting device
- 83: Expansion pin
- 84: Heating cartridge
- 85: Cooling bore

## Claims

1. Method of producing and/or processing as well as winding an endless material of thermoplastic plastic (for example, foil, film, slit film, yarn) to a package with output of a quality signal as a function of a continuously measured process parameter, characterized in that a parameter of state of the package is measured and used as process parameter of the endless material, that the process parameter or a value derived therefrom is compared with a predetermined representative value (desired value) or its tolerance range, and that the quality signal of the quality of the endless material is output in the event of an unacceptable deviation of the parameter of state or derived value from the desired value.

2. Method as in claim 1,
characterized in that
the time-dependent course of the parameter of state over the entire winding cycle or predetermined time intervals of the winding cycle is picked up and compared with a predetermined representative curve or its tolerance range (desired course), and the quality signal is output in the event of a deviation from the desired course.

3. Method as in claim 1,
characterized in that
the parameter of state is determined a predetermined instant of the winding cycle and compared with a predetermined representative value or tolerance zone (desired value), and the quality signal is output in the event of a deviation of the representative value from the desired value.

4. Method as in claim 1,
characterized in that
at the beginning and at the end of a predetermined time interval of the winding cycle, the parameter of state of the package is measured, and the increase of the parameter of state of the time interval is determined and compared with a representative value or tolerance range (desired value) of the increase of the parameter of state, and the quality signal is output in the event of a deviation from the desired value.

5. Method as in claim 1,
characterized in that
the course of increase of the parameter of state per unit time is determined over the entire winding cycle or over a predetermined time interval of the winding cycle and compared with a predetermined representative course or tolerance range (desired value), and the quality signal is output in the event of a deviation from the desired value.

6. Method as in claim 1,
characterized in that
during the winding cycle or a predetermined time interval of the winding cycle one of the coefficients of the time function of the parameter of state is determined and compared with a predetermined representative value or tolerance range (desired value), and the quality signal is output in the event of a deviation from the desired value.

7. Method as in claim 1,
characterized in that
in a multiposition textile machine the parameter of state of the package of each winding position is continuously measured and a mean value is formed from the measured parameter of state of the package or values derived therefrom, and that the parameter of state or the value derived therefrom of each winding position is compared continuously or at certain instants with the mean value or a tolerance range predetermined to this end, and the quality signal is output in the event of a deviation.

8. Method as in one of the foregoing claims,
characterized in that
the parameter of state of the package is its diameter.

9. A method according to one of the foregoing claims,
characterized in that
for drawing results from the measurement of a diameter the diameter is continuously squared and the first mathematical derivative of the square is formed and continuously controllled.

10. Method as in one of the foregoing claims 1-7,
characterized in that
in the production and takeup of films or foils the parameter of state is the actual thickness of the film/foil advancing to the package.

11. Method as in one of the foregoing claims 1-7,
characterized in that
in the production and winding of a yarn to a cross-wound package the parameter of state is the denier of the yarn advancing to the package.

12. Method as in claim 10 or 11,
characterized in that
the thickness or denier is measured by measuring the length of the endless material advancing to the package and the weight of the package.

13. Method as in one of claims 1-7,
characterized in that
the parameter of state is the quantity or weight of the fluid which adheres to the yarn and the package (spin finish application).

14. Method as in claim 13,
characterized in that
the spin finish application is measured by measuring the throughput on the thermoplastic material during the winding cycle or the throughput per unit time and the increase in weight of the package per unit time.

15. Method as in one of claims 1-7,
characterized by
the combination of claims 11 and 12 or claims 11 and 13, the denier or the thickness being measured after removal of the spin finish application.

16. Method as in one of claims 1-7,
characterized in that
the parameter of state is the density or the winding factor (crossing angle) of the package.

17. Method as in claim 16,
characterized in that
the density of the package is measured by measuring the package diameter and the weight.

18. Method as in one of claims 1-7,
characterized in that
the parameter of state is the weight.

19. Method as in one of claims 12, 14, 15, 17, and 18,
characterized in that
the weight is determined by the deformation of the spindle or spindle bearing.

20. Method is in one of claims 12, 14, 15, 17, and 18,
characterized in that
the weight of the package is measured by measuring the weight of the takeup machine.

21. Method as in claim 8 or 17,
characterized in that
the package diameter is determined by measuring the surface speed and the rotational speed of the package.

22. Method as in claim 21,
characterized in that
the surface speed is determined in that the surface of the package is scanned by means of a freely rotatable measuring roll, and its rotational speed is measured.

23. Method as in one of the foregoing claims,
characterized in that
the quality signal is output for identifying the quality of the package.

24. Method as in one of the foregoing claims,
characterized in that
the quality signal is output for monitoring and interrupting the process, if need arises.

25. Method as in one of claims 1-22,
characterized in that
the quality signal is output for controlling a process parameter of the process.

26. Method as in claim 25,
characterized in that
the thermoplastic material is supplied by means of a pump or an extruder to a die, and the rotational speed of the pump or extruder is controlled as a function of the quality signal.

27. Method as in claim 25,
characterized in that
the thermoplastic material is supplied to the die in a plasticized condition, and that the heat supply to the thermoplastic material is controlled as a function of the quality signal.

28. Method as in claim 25,
characterized in that
the thermoplastic material is extruded from the die in a molten condition and cooled upon leaving the die as a function of the quality signal.

29. Method as in claim 28,
characterized in that
the thermoplastic material is extruded in molten condition as a strand of filaments or strand of slit film from a plurality of nozzle holes which are arranged in a nozzle plate, that each strand of filaments or slit film is processed to a yarn and wound on its own package, and that each strand of filaments or slit film is cooled upon leaving the spinneret as a function of the quality signal.

30. Method as in claim 25 for the production of a foil or film,
characterized in that
the thermoplastic material is extruded from a wide-slot die, and that the width of the die orifice is controllable as a function of the quality signal.

31. Method as in claim 30,
characterized in that
the foil web exiting from the wide-slot die is cut to tapes, and that after drawing each slit-film tape is wound to a package, and that the course of the orifice width is controlled over the width of the wide-slot die as a function of the quality signal associated with each package.

32. Takeup machine for a continuously advancing endless material of a thermoplastic substance comprising a winding spindle for receiving and rotatably supporting a package, characterized in that the winding spindle (34) is connected with a measuring device (52) for determining the weight as a process parameter of the endless material whereby the process parameter of the endless material or a value derived therefrom is compared with a predetermined representative value (desired value) or its tolerance range and a quality signal of the quality of the endless material is output in the event of an unacceptable deviation of the parameter of state or derived value from the desired value.

33. Takeup machine for a continuously advancing endless material of a thermoplastic substance comprising a winding spindle for receiving and rotatably supporting packages, characterized in that the takeup machine is connected with a measuring device (52) for determining its change of weight as a process parameter of the endless material whereby the process parameter of the endless material or a value derived therefrom is compared with a predetermined representative value (desired value) or its tolerance range and a quality signal of the quality of the endless material is output in the event of an unacceptable deviation of the parameter of state or derived value from the desired value.

34. Takeup machine as in claim 32 or 33,
characterized in that
the winding head, consisting of winding spindle (34), yarn traversing mechanism (27), contact roll (28), and drive motor (36) is rotatably supported along the line of its center of gravity in a machine frame (62), and adapted to pivot about the axis of rotation of a swing support (60).

35. Takeup machine as in claims 34,
characterized in that
the measuring device (52) for determining the weight or for determining the change in weight of the winding spindle (34) is stationarily mounted on machine frame (62) which is used for the suspension of the swing support (60).

36. Spinning system for producing and/or processing as well as winding endless filaments or monofilaments,
characterized in that it comprises a takeup machine as in claims 32 to 35,
said takeup machine being provided with winding heads (74) which comprise a weight sensor (52) for determining the weight or the change of weight of the winding spindle (34) and the package or packages (33) placed thereon.

37. Slit film production line for producing/and or processing as well as winding slit-film tapes cut from a film web,
characterized in that it comprises a takeup machine according to one of claims 32 to 35, said takeup machine being provided with winding heads (74) which comprise a weight sensor (52) for determining the weight or change in weight of the winding spindle (34) and the package or packages (33) placed thereon, and that the [weights] determined by the weight sensors (52) are processed in a central computer unit (40) and compared with desired values, and that the quality signals output by the computer unit (40) are used for controlling the process (49, 65, 67).

## Patentansprüche

1. Verfahren zur Herstellung und/oder Bearbeitung sowie Aufwickeln eines Endlosmaterials aus thermoplastischem Kunststoff (z.B. Folie, Film, Bändchen, Faden) auf eine Spule mit Ausgabe eines Qualitätssignals in Abhängigkeit von einem kontinuierlich gemessenen Verfahrensparameter, dadurch gekennzeichnet, daß ein Zustandsparameter der Spule gemessen und als Verfahrensparameter des Endlosmaterials eingesetzt wird, daß der Verfahrensparameter oder der davon abgeleitete Wert mit einem vorgegebenen Repräsentativwert (Sollwert) oder dessen Toleranzvergleich verglichen wird, und daß das Qualitätssignal der Qualität des Endlosmaterials bei unzulässiger Abweichung des Zustandsparameters oder des abgeleiteten Wertes vom Sollwert ausgegeben wird.

2. Verfahren nach Anspruch 1, dadurch gekennzeichnet, daß der zeitabhängige Verlauf des Zustandsparameters über die gesamte Spulreise oder vorbestimmte Zeitspannen der Spulreise aufgenommen und mit einer vorgegebenen Repräsentativkurve oder deren Toleranzband (Sollverlauf) verglichen und bei Abweichung von dem Sollverlauf das Qualitätssignal ausgegeben wird.

3. Verfahren nach Anspruch 1, dadurch gekennzeichnet, daß der Zustandsparameter zu einem vorgegebenen Zeitpunkt der Spulreise ermittelt und mit einem vorgegebenen Repräsentativwert oder Toleranzfeld (Sollwert) verglichen und bei Abweichung des Repräsentativwertes vom Sollwert das Qualitätssignal ausgegeben wird.

4. Verfahren nach Anspruch 1, dadurch gekennzeichnet, daß zu Beginn und zum Ende eines vorgegebenen Zeitabschnittes der Spulreise der Zustandsparameter der Spule gemessen und die Zunahme des Zustandsparameters des Zeitabschnittes ermittelt und mit einem Repräsentativwert oder Toleranzbereich (Sollwert) der Zunahme des Zustandsparameters verglichen und bei Abweichung von dem Sollwert das Qualitätssignal ausgegeben wird.

5. Verfahren nach Anspruch 1, dadurch gekennzeichnet, daß über die gesamte Spulreise oder über einen vorgegebenen Zeitabschnitt der Spulreise der Verlauf der Zunahme des Zustandsparameters pro Zeiteinheit ermittelt und mit einem vorgegebenen Repräsentativverlauf oder Toleranzband (Sollwert) verglichen und bei Abweichung von dem Sollwert das Qualitätssignal ausgegeben wird.

6. Verfahren nach Anspruch 1, dadurch gekennzeichnet, daß während der Spulreise oder eines vorgegebenen Zeitabschnittes der Spulreise einer der Koeffizienten der zeitlichen Funktion des Zustandsparameters ermittelt und mit einem vorgegebenen Repräsentativwert oder Toleranzbereich (Sollwert) verglichen und bei Abweichung von dem Sollwert das Qualitätssignal ausgegeben wird.

7. Verfahren nach Anspruch 1, dadurch gekennzeichnet, daß in einer mehrstelligen Textilmaschine der Zustandsparameter der Spule jeder Aufwikkelstelle kontinuierlich gemessen und aus dem gemessenen Zustandsparameter der Spule oder davon abgeleiteten Werten ein Mittelwert gebildet wird und daß der Zustandsparameter oder der daraus abgeleitete Wert jeder Aufwickelstelle kontinuierlich oder zu bestimmten Zeitpunkten mit dem Mittelwert oder einem dazu vorgegebenen Toleranzband verglichen und bei Abweichung von dem Sollwert das Qualitätssignal ausgegeben wird.

8. Verfahren nach einem der vorangegangenen Ansprüche, dadurch gekennzeichnet, daß der Zustandsparameter der Spule ihr Durchmesser ist.

9. Verfahren nach einem der vorangegangenen Ansprüche, dadurch gekennzeichnet, daß zum Erzielen von Ergebnissen aus der Messung eines Durchmessers der Durchmesser kontinuierlich quadriert wird und die erste mathematische Ableitung der Quadratzahl gebildet und kontinuierlich kontrolliert wird.

10. Verfahren nach einem der vorangegangenen Ansprüche 1-7, dadurch gekennzeichnet, daß bei der Herstellung und Aufwicklung von Filmen oder Folien der Zustandsparameter die aktuelle Dicke des der Spule zulaufenden Films/Folie ist.

11. Verfahren nach einem der vorangegangenen Ansprüche 1-7, dadurch gekennzeichnet, daß bei der Herstellung und Aufwicklung eines Fadens zu einer Kreuzspule der Zustandsparameter die Fadenstärke des der Spule zulaufenden Fadens ist.

12. Verfahren nach Anspruch 10 oder 11, dadurch gekennzeichnet, daß die Dicke oder Fadenstärke durch Messung der Lauflänge des der Spule zulaufenden Endlosmaterials und des Gewichtes der Spule gemessen wird.

13. Verfahren nach einem der Ansprüche 1-7, dadurch gekennzeichnet, daß der Zustandsparameter die Menge oder das Gewicht der Flüssigkeit ist, die an dem Faden und der Spule haftet (Präparationsauftrag).

14. Verfahren nach Anspruch 13, dadurch gekennzeichnet, daß der Präparationsauftrag durch Messung des Durchsatzes an dem thermoplastischen Material während der Spulreise oder des Durchsatzes pro Zeiteinheit und des Gewichtszuwachses der Spule pro Zeiteinheit gemessen wird.

15. Verfahren nach einem der Ansprüche 1-7, gekennzeichnet durch die Kombination der Ansprüche 11 und 12 oder der Ansprüche 11 und 13, wobei die Fadenstärke oder die Dicke nach Abzug des Präparationsauftrages gemessen wird.

16. Verfahren nach einem der Ansprüche 1-7, dadurch gekennzeichnet, daß der Zustandsparameter die Dichte oder der Wickelfaktor (Kreuzungswinkel) der Spule ist.

17. Verfahren nach Anspruch 16, dadurch gekennzeichnet, daß die Dichte der Spule durch die Messung des Spulendurchmessers und des Gewichtes gemessen wird.

18. Verfahren nach einem der Ansprüche 1-7, dadurch gekennzeichnet, daß der Zustandsparameter das Gewicht ist.

19. Verfahren nach einem der Ansprüche 12, 14, 15, 17 und 18, dadurch gekennzeichnet, daß das Gewicht durch Verformung der Spindel oder der Spindellagerung ermittelt wird.

20. Verfahren nach einem der Ansprüche 12, 14, 15, 17 und 18, dadurch gekennzeichnet, daß das Gewicht der Spindel durch Messung des Gewichts der Aufwickelmaschine ermittelt wird.

21. Verfahren nach Anspruch 8 oder 17, dadurch gekennzeichnet, daß der Spulendurchmesser durch Messung der Oberflächengeschwindigkeit und der Drehzahl der Spule ermittelt wird.

22. Verfahren nach Anspruch 21, dadurch gekennzeichnet, daß die Oberflächengeschwindigkeit ermittelt wird, indem die Oberfläche der Spule mittels einer frei drehenden Meßwalze abgetastet und deren Drehzahl gemessen wird.

23. Verfahren nach einem der vorangegangenen Ansprüche, dadurch gekennzeichnet, daß das Qualitätssignal zur Qualitätskennzeichnung der Spule ausgegeben wird.

24. Verfahren nach einem der vorangegangenen Ansprüche, dadurch gekennzeichnet, daß das Qualitätssignal zur Überwachung und zur Unterbrechung des Verfahrens ausgegeben wird.

25. Verfahren nach einem der Ansprüche 1-22, dadurch gekennzeichnet, daß das Qualitätssignal zur Steuerung eines Prozeßparameters des Verfahrens ausgegeben wird.

26. Verfahren nach Anspruch 25, dadurch gekennzeichnet, daß das thermoplastische Material mittels einer Pumpe oder eines Extruders einer Düse zugeführt und die Drehzahl der Pumpe oder des Extruders in Abhängigkeit vom Qualitätssignal gesteuert wird.

27. Verfahren nach Anspruch 25, dadurch gekennzeichnet, daß das thermoplastische Material dem Werkzeug in plastifiziertem Zustand zugeführt wird und daß die Wärmezufuhr zu dem thermoplastischen Material in Abhängigkeit von dem Qualitätssignal gesteuert wird.

28. Verfahren nach Anspruch 25, dadurch gekennzeichnet, daß das thermoplastische Material vom Werkzeug in geschmolzenem Zustand extrudiert wird und nach Verlassen der Düse in Abhängigkeit von dem Qualitätssignal gekühlt wird.

29. Verfahren nach Anspruch 28, dadurch gekennzeichnet, daß das thermoplastische Material in geschmolzenem Zustand aus einer Vielzahl von Düsenlöchern, die in einer Düsenplatte angeordnet sind, als Fadenstrang oder Bändchenstrang extrudiert wird, daß jeder Faden- oder Bändchenstrang zu einem Faden bearbeitet und auf seine eigene Spule aufgespult wird und daß jeder Faden- oder Bändchenstrang nach Verlassen der Spinnplatte in Abhängigkeit von dem Qualitätssignal gekühlt wird.

30. Verfahren nach Anspruch 25 zur Herstellung einer Folie oder eines Films, dadurch gekennzeichnet, daß das thermoplastische Material von einem Werkzeug mit breiten Schlitzen extrudiert wird und daß die Weite der Werkzeugöffnung in Abhängigkeit vom Qualitätssignal steuerbar ist.

31. Verfahren nach Anspruch 30, dadurch gekennzeichnet, daß der aus der Breitschlitzdüse austretende Folienstrang zu Bändern zerschnitten wird, daß jedes Bändchen nach der Verstreckung zu einer Spule aufgewickelt wird und daß der Verlauf der Spaltweite über die Breite des Breitschlitzwerkzeugs in Abhängigkeit von dem jeder Spule zugeordneten Qualitätssignal gesteuert wird.

32. Aufwickelmaschine für ein kontinuierlich anfallendes Endlosmaterial aus einer thermoplastischen Substanz, die eine Spulspindel zur Aufnahme und zur drehbaren Lagerung einer Spule umfaßt, dadurch gekennzeichnet, daß die Spulspindel (34) mit einer Meßvorrichtung (52) zur Bestimmung des Gewichts als Prozeßparameter des Endlosmaterials verbunden ist, wobei der Prozeßparameter des Endlosmaterials oder ein davon abgeleiteter Wert mit einem vorgegebenen Repräsentativwert (Sollwert) oder seinem Toleranzbereich verglichen wird und ein Qualitätssignal der Qualität des Endlosmaterials bei unzulässiger Abweichung des Zustandsparameters oder des abgeleiteten Wertes vom Sollwert ausgegeben wird.

33. Aufwickelmaschine für ein kontinuierlich anfallendes Endlosmaterial aus einer thermoplastischen Substanz, die eine Spulspindel zur Aufnahme und drehbaren Lagerung von Spindeln umfaßt, dadurch gekennzeichnet, daß die Aufwickelmaschine mit einer Meßvorrichtung (52) zur Bestimmung der Gewichtsänderung als Prozeßparameter des Endlosmaterials verbunden ist, wobei der Prozeßparameter des Endlosmaterials oder ein davon abgeleiteter Wert mit einem vorgegebenen Repräsentativwert (Sollwert) oder seinem Toleranzbereich verglichen wird und ein Qualitätssignal der Qualität des Endlosmaterials bei unzulässiger Abweichung des Zustandsparameters oder des abgeleiteten Wertes vom Sollwert ausgegeben wird.

34. Aufwickelmaschine nach Anspruch 32 oder 33, dadurch gekennzeichnet, daß der Spulkopf, der eine Spulspindel (34), einen Changiermechanismus (27), eine Kontaktwalze (28) und einen Antriebsmotor (36) umfaßt, auf seiner Schwerpunktlinie in einem Maschinenrahmen (62) drehbar gelagert ist, so daß er um die Rotationsachse eines Kipplagers (60) schwenkbar ist.

35. Aufwickelmaschine nach Anspruch 34, dadurch gekennzeichnet, daß die Meßvorrichtung (52) zur Bestimmung des Gewichts- oder der Gewichtsänderung der Spulspindel (34) ortsfest auf dem Maschinenrahmen (62) montiert ist, der zur Aufhängung des Kipplagers (60) dient.

36. Spinnsystem zur Herstellung und/oder Bearbeitung sowie zum Aufwickeln von Endlosfäden oder Monofilen, dadurch gekennzeichnet, daß es eine Aufwickelmaschine nach den Ansprüchen 32 bis 35 umfaßt, wobei die Aufwickelmaschine mit Spulköpfen (74) versehen ist, die einen Gewichtssensor (52) zur Bestimmung des Gewichts oder der Gewichtsänderung der Spulspindel (34) und die auf letzterer angeordneten Spule(n) (33) umfassen.

37. Folienbändchenanlage zur Herstellung und/oder Bearbeitung sowie zum Aufwickeln von aus einer Folienbahn geschnittenen Folienbändchen, dadurch gekennzeichnet, daß die Folienbändchenanlage eine Aufwickelmaschine nach einem der Ansprüche 32 bis 35 umfaßt, wobei die Aufwikkelmaschine mit Spulköpfen (74) versehen ist, die einen Gewichtssensor (52) zur Bestimmung des Gewichts oder der Gewichtsänderung der Spulspindel (34) und die auf letzterer angeordneten Spule(n) (33) umfassen, wobei die von den Gewichtssensoren (52) ermittelten Gewichte in einer zentralen Rechnereinheit (40) verarbeitet und mit Sollwerten verglichen werden und wobei die von der Rechnereinheit (40) ausgegebenen Qualitätssignale zur Steuerung des Prozesses (49, 65, 67) verwendet werden.

## Revendications

1. Procédé de production et/ou de traitement d'un bobinage d'une matière thermoplastique sans fin (par exemple une feuille, un film, un film fendu, un fil) pour former une bobine, avec délivrance d'un signal de qualité en fonction d'un paramètre de traitement mesuré en continu, caractérisé en ce qu'un paramètre d'état de la bobine est mesuré et utilisé en tant que paramètre de traitement de la matière sans fin, que le paramètre de traitement ou une valeur, qui en est dérivée, est comparé à une valeur représentation prédéterminée (valeur désirée) ou à sa plage de tolérance, et que le signal de la qualité de la matière sans fin est délivré dans le cas d'un écart inacceptable du paramètre d'état ou de la valeur dérivée par rapport à la valeur désirée.

2. Procédé selon la revendication 1, caractérisé en ce que
la variation, qui dépend du temps, du paramètre d'état sur l'ensemble du cycle de bobinage ou sur des intervalles de temps prédéterminés du cycle de bobinage est enregistrée et comparée à une courbe représentative prédéterminée ou à sa plage de tolérance (variation désirée) et le signal de qualité est délivré dans le cas d'un écart par rapport à la variation désirée.

3. Procédé selon la revendication 1, caractérisé en ce que
le paramètre d'état est déterminé à un instant prédéterminé du cycle de bobinage et comparé à une valeur représentative prédéterminée ou à une plage de tolérance (valeur désirée) et le signal de qualité est délivré dans le cas d'un écart de la valeur représentative par rapport à la valeur désirée.

4. Procédé selon la revendication 1, caractérisé en ce que
au début et à la fin d'un intervalle de temps prédéterminé du cycle de bobinage, le paramètre d'état de la bobine est mesuré et l'augmentation du paramètre d'état de l'intervalle de temps est déterminée et est comparée à une valeur représentative ou à une plage de tolérance (valeur désirée) de l'accroissement du paramètre d'état, et le signal de qualité est délivré dans le cas d'un écart par rapport à la valeur désirée.

5. Procédé selon la revendication 1, caractérisé en ce que
la variation de l'accroissement du paramètre d'état par unité de temps est déterminée sur l'ensemble du cycle de bobinage ou sur un intervalle de temps prédéterminé du cycle de bobinage, comparé à une course représentative prédéterminée ou une plage de tolérance (valeur désirée), et le signal de qualité est délivré dans le cas d'un écart par rapport à la valeur désirée.

6. Procédé selon la revendication 1, caractérisé en ce que
pendant le cycle de bobinage ou pendant un intervalle de temps prédéterminé du cycle de bobinage, l'un des coefficients de la fonction temporelle du paramètre d'état est déterminé et est comparé à une valeur représentative prédéterminée ou à une plage de tolérance (valeur désirée), et le signal de qualité est délivré dans le cas d'un écart par rapport à la valeur désirée.

7. Procédé selon la revendication 1, caractérisé en ce que
dans une machine textile à positions multiples, le paramètre d'état de la bobine pour chaque position de bobinage est mesuré continûment et une valeur est formée à partir du paramètre d'état mesuré de la bobine ou de valeurs qui en sont dérivées, et que le paramètre d'état ou la valeur, qui en est dérivée, de chaque position de bobine est comparé continûment ou à certains instants à la valeur moyenne ou à une plage de tolérance prédéterminée à cet effet, et le signal de qualité est délivré dans le cas d'un écart.

8. Procédé selon l'une des revendications précédentes, caractérisé en ce que
le paramètre d'état de la bobine est son diamètre.

9. Procédé selon l'une des revendications précédentes, caractérisé en ce que
pour tirer des résultats de la mesure d'un diamètre, le diamètre est en permanence élevé au carré et la dérivée mathématique première du carré est formée et est contrôlée d'une manière continue.

10. Procédé selon l'une des revendications précédentes 1-7, caractérisé en ce que
lors de la fabrication et du bobinage de films ou de feuilles, le paramètre d'état est l'épaisseur réelle du film/de la feuille qui avance en direction de la bobine.

11. Procédé selon l'une des revendications précédentes 1-7, caractérisé en ce que
lors de la production et du bobinage d'un fil sur une bobine à bobinage croisé, le paramètre d'état est le denier du fil qui avance jusqu'à la bobine.

12. Procédé selon la revendication 10 ou 11, caractérisé en ce que
l'épaisseur ou le denier est mesuré par mesure de la longueur de la matière sans fin, qui avance jusqu'à la bobine, et du poids de la bobine.

13. Procédé selon l'une des revendications précédentes 1-7, caractérisé en ce que
le paramètre d'état est la quantité ou le poids du fluide, qui adhère au fil et à la bobine (application d'un adjuvent de filage).

14. Procédé selon la revendication 13, caractérisé en ce que
l'application d'un adjuvant de filage est mesurée au moyen de la mesure du débit de sortie de la matière thermoplastique pendant le cycle de bobinage ou le débit par unité de temps et l'accroissement du poids de la bobine par unité de temps.

15. Procédé selon l'une des revendications 1-7, caractérisé par
la combinaison des revendications 11 et 12 ou des revendications 11 et 13, le denier ou l'épaisseur étant mesuré après suppression de l'application d'un adjuvant de filage.

16. Procédé selon l'une des revendications 1-7, caractérisé en ce que
le paramètre d'état est la densité ou le facteur de bobinage (angle de croisement) de la bobine.

17. Procédé selon la revendication 16, caractérisé en ce que
la densité de la bobine est mesurée par mesure du diamètre de la bobine et du poids.

18. Procédé selon l'une des revendications 1-7, caractérisé en ce que
le paramètre d'état est le poids.

19. Procédé selon l'une des revendications 12, 14, 15, 17, 18, caractérisé en ce que
le poids est déterminé par la déformation de la broche ou du palier de broche.

20. Procédé selon l'une des revendications 12, 14, 15, 16, 17 et 18, caractérisé en ce que
le poids de la bobine est mesuré par mesure du poids de la machine d'enroulement.

21. Procédé selon la revendication 10 ou 17, caractérisé en ce que
le diamètre de la bobine est déterminé par mesure de la vitesse superficielle et la vitesse de rotation de la bobine.

22. Procédé selon la revendication 21, caractérisé en ce que
la vitesse superficielle est déterminée par le fait que la surface de la bobine est scannée au moyen d'un rouleau de mesure pouvant tourner librement, et sa vitesse de rotation est mesurée.

23. Procédé selon l'une des revendications précédentes, caractérisé en ce que
le signal de qualité est délivré pour identifier la qualité de la bobine.

24. Procédé selon l'une des revendications précédentes, caractérisé en ce que
le signal de qualité est délivré pour le contrôle et l'interruption du processus, si cela s'avère nécessaire.

25. Procédé selon l'une des revendications 1-22, caractérisé en ce que
le signal de qualité est délivré pour la commande d'un paramètre du traitement.

26. Procédé selon la revendication 25, caractérisé en ce que
la matière thermoplastique est délivrée au moyen d'une pompe ou d'une extrudeuse à une matrice, et la vitesse de rotation de la pompe ou de l'extrudeuse est commandée en fonction du signal de qualité.

27. Procédé selon la revendication 25, caractérisé en ce que
la matière thermoplastique est envoyée à la matrice dans un état plastifié, et que l'application de chaleur à la matière plastique est commandée en fonction du signal de qualité.

28. Procédé selon la revendication 25, caractérisé en ce que
la matière thermoplastique est extrudée de la matrice dans un état fondu et est refroidie lorsqu'elle quitte la matrice en fonction du signal de qualité.

29. Procédé selon la revendication 28, caractérisé en ce que
la matière thermoplastique est extrudée à l'état fondu sous la forme d'un toron de filaments ou d'un toron d'un film fendu arrivant d'une pluralité de trous de buses, qui sont disposés dans une plaque à buses, que chaque toron de filaments ou de film fendu est traité comme un fil et est enroulé sur son propre bobine et que chaque toron de filaments ou de film fendu est refroidi lorsqu'il quitte la filière, en fonction du signal de qualité.

30. Procédé selon la revendication 25 pour la fabrication d'une feuille ou d'un film, caractérisé en ce que
la matière thermoplastique est extrudée d'une filière à fente large et que la largeur de l'orifice de filière peut être commandée en fonction du signal de qualité.

31. Procédé selon la revendication 30, caractérisé en ce que
la bande en forme de feuille, qui sort de la matrice à fente large, est découpée en rubans et qu'après étirage, chaque ruban de film fendu est enroulé pour former une bobine et que l'allure de la largeur de l'orifice est commandée sur la largeur de la matrice à fente large en fonction du signal de qualité associé à chaque bobine.

32. Machine d'enroulement pour une matière sans fin, qui avance en continu et est formée d'une substance thermoplastique, comprenant une broche de bobinage servant à recevoir et supporter avec possibilité de rotation une bobine, caractérisée en ce que la broche de bobinage (34) est raccordée à un dispositif de mesure (52) servant à déterminer le poids en tant que paramètre de traitement de la matière sans fin, grâce à quoi le paramètre de traitement de la matière sans fin ou une valeur dérivée de ce paramètre est comparé à une valeur représentative prédéterminée (valeur désirée) ou à sa plage de tolérance, et un signal de la qualité de la matière sans fin est délivré dans le cas d'un écart inadmissible du paramètre d'état ou d'une valeur dérivée de la valeur désirée.

33. Machine d'enroulement pour une matière sans fin, qui avance en continu et est formée d'une substance thermoplastique, comprenant une broche de bobinage servant à recevoir et supporter avec possibilité de rotation une bobine, caractérisée en ce que la broche de bobinage est raccordée à un dispositif de mesure (52) pour déterminer la variation de son poids en tant que paramètre de traitement de la matière sans fin, ce qui a pour effet que le paramètre de traitement de la matière sans fin ou une valeur dérivée de ce paramètre est comparé à une valeur représentative prédéterminée (valeur désirée) ou à sa plage de tolérance, et un signal de la qualité de la matière sans fin est délivré dans le cas d'un écart inadmissible du paramètre d'état ou d'une valeur dérivée de la valeur désirée.

34. Machine d'enroulement selon la revendication 32 ou 33, caractérisée en ce que
la tête de bobinage, qui est constituée par la broche de bobinage (34), par un mécanisme (27) de déplacement du fil, par un rouleau de contact (28) et par un moteur d'entraînement (36), est supportée de manière à pouvoir tourner le long de l'axe passant par son centre de gravité dans un châssis (62) de la machine et est adaptée de manière à pouvoir pivoter autour de l'axe de rotation d'un support oscillant (60).

35. Machine d'enroulement selon la revendication 34, caractérisée en ce que
le dispositif de mesure (52) servant à déterminer le poids ou à déterminer la variation du poids de la broche d'enroulement (34) est monté de façon fixe sur le châssis (62) de la machine, qui est utilisé pour la suspension du support oscillant (60).

36. Système d'enroulement pour la production et/ou le traitement ainsi que le bobinage de filaments sans fin ou de monofilaments, caractérisé en ce
qu'il comporte une machine d'enroulement selon les revendications 32 à 35, ladite machine d'enroulement étant pourvue de têtes de bobinage (74), qui comprennent un capteur de poids (52) pour déterminer le poids ou la variation du poids de la broche de bobinage (34) et de la bobine ou des bobines (33) placées sur cette broche.

37. Chaîne de production d'un film fendu pour la production et/ou le traitement ainsi que le bobinage de rubans de film fendu découpés à partir d'une bande de film, caractérisée en ce
qu'elle comprend une machine d'enroulement selon l'une des revendications 32 à 35, ladite machine d'enroulement étant pourvue de têtes de bobinage (74), qui comprennent un capteur de poids (52) pour déterminer le poids ou la variation du poids de la broche de bobinage (34) et de la bobine ou des bobines (33) placées sur cette broche, et
que les poids déterminés par les capteurs de poids (52) sont traités dans une unité centrale d'ordinateur (40) et sont comparés à des valeurs désirées, et que les signaux de qualité délivrés par l'unité d'ordinateur (40) sont utilisés pour commander le processus (49, 65, 67).
